# EUROPÄISCHE PATENTSCHRIFT

(11) **EP 0 313 820 B1**
(45) Veröffentlichungstag und Bekanntmachung des Hinweises auf die Patenterteilung: **21.04.1993**
(21) Anmeldenummer: 88115631.9
(22) Anmeldetag: 23.09.1988
(51) Int. Cl.: A61B 17/28, A61B 17/32, B25B 7/00, B25B 13/26, B25B 17/02

(54) **Zange, insbesondere Hakenstanze**
Forceps, in particular claw-shaped cutters
Pince, en particulier poinçonneuse à crochet

(30) Priorität: 26.10.1987 DE 3736150
(43) Veröffentlichungstag der Anmeldung: 03.05.1989
(73) Patentinhaber: Richard Wolf GmbH, 75438 Knittlingen (DE)
(72) Erfinder: Falk, Ernst, D-7137 Sternenfels-Diefenbach (DE)
(74) Vertreter: Wilcken, Thomas, Dipl.-Ing.

(56) Entgegenhaltungen:
- DE-U- 8 316 034
- US-A- 2 518 994

## Beschreibung

Die Erfindung geht aus von einer Zange, insbesondere einer Hakenstanze, nach dem Oberbegriff des Patentanspruches 1. Solche z.B. im DE-GM 83 16 034 beschriebenen Hakenstanzen mit einem feststehenden und einem verschwenkbaren Schenkel des Zangenmaules sind so ausgebildet, daß die Schließlage und die Offenlage durch Anschläge auf der den verschwenkbaren Maulschenkel betätigenden Schub- und Zugstange begrenzt werden, der in den beiden Endlagen des schwenkbaren Maulschenkels gegen feststehende Anschläge zur Anlage kommt.

Solche Anschläge sind nur brauchbar, wenn die Zange und deren Schaftdurchmesser genügend große Abmessungen aufweisen, da dann die Anschläge eine sichere Begrenzung der Offen- und Schließlage des Zangenmaules gewährleisten.

Die Aufgabe der Erfindung besteht darin, sowohl für Zangen oder Hakenstanzen mit größeren als auch kleinsten Abmessungen, insbesondere mit Schaftdurchmessern zwischen etwa 1,5 bis 2,5 mm , die Anschläge so zu bemessen, anzuordnen und konstruktiv auszubilden, daß sie eine sichere Begrenzung der Offen- und Schließlage des Zangenmaules gewährleisten.

Die Aufgabe wird bei Zangen, Hakenstanzen oder dergleichen nach dem Oberbegriff des Anspruches 1 durch die kennzeichnenden Merkmale dieses Anspruches 1 gelöst. Weitere vorteilhafte Merkmale ergeben sich aus den Ansprüchen 2 bis 4.

Durch die Verlegung der Anschläge in den Bereich des Zangenmaulteiles und deren konstruktive Gestaltung sind diese auch bei kleinsten Zangendurchmessungen sehr stabil und bilden somit eine sichere Begrenzung für die Endlagen des schwenkbaren Maulschenkels, ohne daß der Patient in unbeabsichtigter Weise verletzt oder der Zangenmaulschenkel beschädigt wird.

Die Erfindung wird nachstehend anhand der Zeichnungen erläutert. Es zeigt:
- Figur 1: die Seitenansicht einer Hakenstanze mit vergrößertem distalen Ende,
- Figur 2: einen Längsschnitt durch das distale Ende der Hakenstanze nach Figur 1 mit geöffnetem Zangenmaul in starker Vergrößerung und
- Figur 3: einen der Figur 2 entsprechenden Längsschnitt mit geschlossenem Zangenmaul.

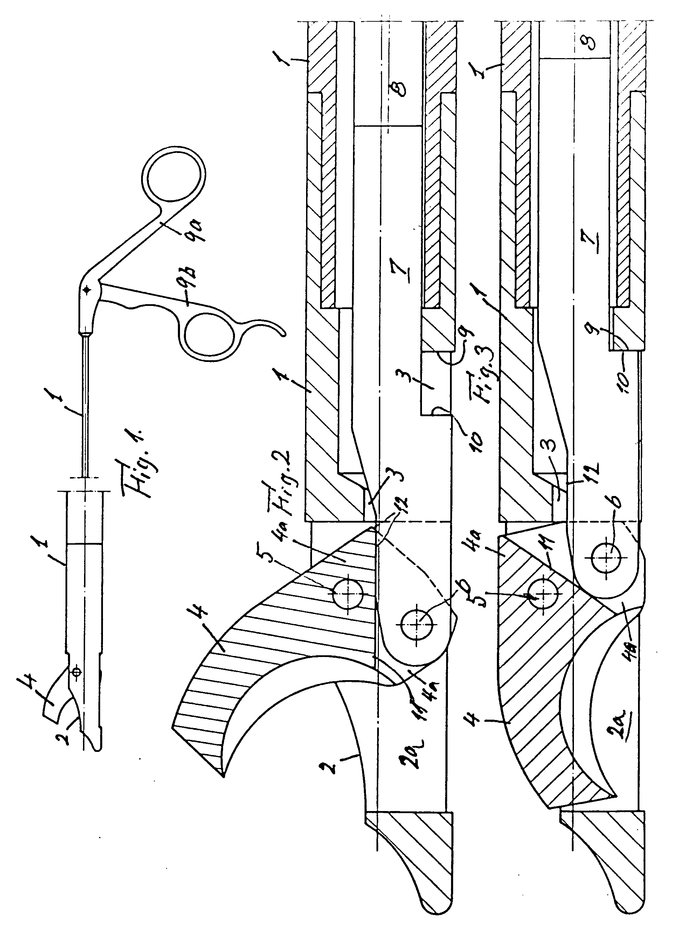

Die Zange oder Hakenstanze nach der Erfindung besteht aus einem Schaft 1 mit einem distalen, den feststehenden Schneidschenkel 2 des Zangenmaules bildenden Durchbruch 2a, an den sich proximalseitig ein unterer Längsschlitz 3 des Schaftes anschließt. Im Durchbruch 2a ist der bewegliche, doppelarmige Maulschenkel 4um den Querzapfen 5 verschwenkbar gelagert. Zu diesem Zweck greift an den Querzapfen 6 des Schenkelarmes 4a das Ende 7 einer Schub- und Zugstange 8 an, die durch den relativ zu dem mit dem Schaft 1 starr verbundenen Scherengriff 9a bewegbaren Scherengriff 9b zur Verschwenkung des Maulschenkels 4 längsbeweglich ist. Die Schubstange 8 greift am distalen Ende mit dem flach ausgebildeten Teil 7 bei 6 an dem Schenkelarm 4a an, wobei der Teil 7 in dem Schaftschlitz 3 geführt ist. Die Verbindung der Teile 7 und 8 oder sonstiger Teile erfolgt durch Laserstrahlverschweißung. Der Schaftschlitz 3 bildet mit seiner proximalen Abschlußkante einen Anschlag 9 und der flache Schubstangenteil 7 ist durch eine untere Abstufung mit einer Stufenfläche 10 versehen, die in der Schließlage des Zangenmaules (Fig. 3) parallel mit der Anschlagfläche 9 in Berührung kommt, derart, daß dadurch die Verschwenkung des Maulschenkels 4 mit der Lage seiner Schneidkante stets innerhalb des Bereiches der Durchbrechung 2a begrenzt wird.

In der Offenlage des Zangenmaules nach Fig. 2 liegt eine proximalseitige Bodenfläche 11 einer gabelförmigen Ausnehmung des Schenkelarmes 4a gegen die distale Oberkante 12 der Schubstange 8.

Wenn die Raumformen und Abmessungen der Zange bzw. Hakenstanze mit einem Schaftdurchmesser zwischen 1, 5 bis 2,5 mm liegen, können der Durchbruch 2a und die flache distale Verlängerung 7 der Schubstange 8 durch das Draht-Erodierverfahren hergestellt werden, während der Längsschlitz 3 für die Führung des flachen Schubstangenendes 7 als auch die für die Lagerung des Zapfens 6 dienende Gabelausnehmung des Schenkelarmes 4a durch das Senk-Erodierverfahren hergestellt werden, wobei die Gabelausnehmung allerdings auch durch spangebende Bearbeitung oder insbesondere durch einen Sägevorgang hergestellt werden kann. Durch diese Verfahren können die beiden Anschlagflächen 9 und 10 absolut planparallel hergestellt werden, so daß weder eine einseitige Abnutzung an diesen Flächen auftreten noch eine Lageänderung des Maulschenkels 4 bei geschlossenem Zangenmaul eintreten kann.

## Patentansprüche

1. Zange, insbesondere Hakenstanze, mit einem am distalen Ende eines Schaftes (1) vorgesehenen Zangenmaul, dessen einer feststehender Schenkel (2) durch das distale, mit einem Durchbruch (2a) versehenen Ende des Schaftes (1) gebildet ist und dessen anderer doppelarmiger Maulschenkel (4) durch einen Scherengriff (9b) und eine angelenkte Schub- und Zugstange (8) um eine Querachse (6) verschwenkbar ist, wobei die Offen- und Schließlage des Zangenmaules durch Anschläge (9,10,11,12) festgelegt ist, dadurch gekennzeichnet, daß in der Schließlage des Zangenmaules gegen eine proximale Abschlußkante (9) eines unteren Längsschlitzes (3) des Schaftes (1)eine untere Abstufung (10) der Zugstange (7,8) anliegt und daß in der Offenstellung eine proximalseitige Stirnfläche (11) des schwenkbaren Maulschenkels (4) gegen die Oberkante (12) am distalen Ende der Schubstange (7,8) liegt.

2. Zange nach Anspruch 1, bei der der Schaft (1) über die axiale Länge des Maules mit einer von oben nach unten durchlaufenden Durchbrechung (2a) für den verschwenkbaren Maulschenkel (4) versehen ist, dadurch gekennzeichnet, daß ein sich an den Schaftdurchbruch (2a) anschließender unterer Schaftschlitz (3) eine distal abgeflachte Verlängerung (7) der Zugstange (8) aufnimmt, welche die untere als Anschlag dienende, eine Abstufungsfläche (10) aufweisende Verlängerung (7) gelenkig mit dem proximalen Arm (4a) des schwenkbaren Maulschenkels (4) verbindet.

3. Zange nach Anspruch 1 oder 2, dadurch gekennzeichnet, daß der Außendurchmesser des Schaftes (1) zwischen 1,5 bis 2,5 mm beträgt.

4. Zange nach den Ansprüchen 1 bis 3, dadurch gekennzeichnet, daß der den feststehenden Schenkel (2) des Zangenmaules bildende distale Schaftdurchbruch (2a) und die flache Verlängerung (7) der Schubstange (8) durch Anwendung des Draht-Erodierverfahrens und der sich an den Durchbruch (2a) des Schaftes (1) anschließende Längsschlitz (3) durch das Senk-Erodierverfahren hergestellt sind.

## Claims

1. Forceps, in particular hook-shaped punch, with a forceps jaw provided at the distal end of a shaft (1), one fixed shank (2) of which is formed by the distal end of the shaft (1), provided with an opening (2a), and the other, double-armed jaw shank (4) of which is pivotable about a transverse axis (6) by a scissor handle (9b) and an articulated push- and pull rod (8), in which the open and closed position of the pincer jaw is fixed by stops (9,10,11,12), characterized in that in the closed position of the pincer jaw, a lower step (10) of the pull rod (7,8) rests against a proximal terminal edge (9) of a lower longitudinal slit (3) of the shaft (1), and that in the open position proximal end face (11) of the orientable jaw shank (4), lies against the upper edge (12) at the distal end of the push rod (7,8).

2. Forceps according to claim 1, in which the shaft (1) is provided over the axial length of the jaw with an opening (2a), running through from top to bottom, for the pivotable jaw shank (4), characterised in that a lower shaft slit (3), joining on to the shaft opening (2a), receives a distally flattened extension (7) of the pull rod (8), which connects articulatedly the lower extension (7), serving as a stop and having a stepped surface (10), with the proximal arm (4a) of the pivotable jaw shank (4).

3. Forceps according to claim 1 or 2, characterised in that the outer diameter of the shaft (1) is between 1.5 and 2.5 mm.

4. Forceps according to claims 1 to 3, characterised in that the distal shaft opening (2a), forming the fixed shank (2) of the forceps jaw, and the flat extension (7) of the push rod (8) are produced by the application of the wire eroding method and the longitudinal slit (3) adjoining the opening (2a) of the shaft (1) is produced by the sink eroding method.

## Revendications

1. Pince, notamment poinçonneuse à crochet, comportant une bouche de pince prévue à l'extrémité distale d'une tige (1) et dont une branche restant fixe (2) est formée par l'extrémité distale de la tige (1), qui est pourvue d'un passage (2a), et dont l'autre branche de bouche à deux bras (4) peut pivoter autour d'un axe transversal (6), par l'intermédiaire d'une poignée de cisaille (9b) et d'une tige articulée de poussée et de traction (8), la position ouverte et la position fermée de la bouche de pince étant déterminées par des butées (9, 10, 11, 12), caractérisée en ce que, lorsque la bouche de pince est fermée, une partie étagée inférieure (10) de la tige de traction (7, 8) s'applique contre un bord proximal de fermeture (9) d'une fente longitudinale inférieure (3) de la tige (1), et que, dans la position ouverte, une surface frontale (11), située du côté proximal, de la branche de bouche pivotante (4) s'applique contre le bord supérieur (12) situé à l'extrémité distale de la tige de poussée (7, 8).

2. Pince selon la revendication 1, dans laquelle la tige (1) est pourvue, sur la longueur axiale de la bouche, d'un passage traversant (2a), qui s'étend de haut en bas, pour la branche de bouche pivotante (4), caractérisée en ce qu'une fente inférieure (3) de tige, qui se raccorde au passage (2a) de tige, accueille un prolongement distal aplati (7) de la tige de traction (8), ce prolongement reliant d'une manière articulée, le prolongement inférieur (7), qui sert de butée de présente une surface étagée (10), avec le bras proximal (4a) de la branche de bouche pivotante (4).

3. Pince selon la revendication 1 ou 2, caractérisée en ce que le diamètre extérieur de la tige (1) est compris entre 1,5 et 2,5 mm.

4. Pince selon les revendications 1 à 3, caractérisée en ce que le passage de tige distal (2a) formant la branche restant fixe (2) de la bouche de pince et le prolongement plat (7) de la tige de poussée (8) sont réalisés par utilisation du procédé d'érosion par fil et que la fente longitudinale (3), qui se raccorde au passage (2a) de la tige (1), est réalisée au moyen du procédé d'érosion en plongée.
